(19) Europäisches Patentamt
European Patent Office
Office européen des brevets

(11) EP 1 961 723 A1

(12) # EUROPEAN PATENT APPLICATION
published in accordance with Art. 158(3) EPC

(43) Date of publication:
**27.08.2008 Bulletin 2008/35**

(21) Application number: **06834110.6**

(22) Date of filing: **06.12.2006**

(51) Int Cl.:
***C07C 27/02*** *(2006.01)* ***B01D 3/14*** *(2006.01)*
***B01D 3/22*** *(2006.01)* ***B01D 3/32*** *(2006.01)*
***C07C 29/128*** *(2006.01)* ***C07C 31/20*** *(2006.01)*
***C07C 68/06*** *(2006.01)* ***C07C 69/96*** *(2006.01)*
***C07B 61/00*** *(2006.01)*

(86) International application number:
**PCT/JP2006/324355**

(87) International publication number:
**WO 2007/069514 (21.06.2007 Gazette 2007/25)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.12.2005 JP 2005360232**

(71) Applicant: **Asahi Kasei Chemicals Corporation**
**Tokyo 100-8440 (JP)**

(72) Inventors:
- **FUKUOKA, Shinsuke**
  **Tokyo 100-8440 (JP)**
- **MIYAJI, Hironori**
  **Tokyo 100-8440 (JP)**
- **HACHIYA, Hiroshi**
  **Tokyo 100-8440 (JP)**
- **MATSUZAKI, Kazuhiko**
  **Tokyo 100-8440 (JP)**

(74) Representative: **Strehl Schübel-Hopf & Partner**
**Maximilianstrasse 54**
**80538 München (DE)**

(54) **PROCESS FOR PRODUCTION OF DIALKYL CARBONATE AND DIOL IN INDUSTRIAL SCALE AND WITH HIGH YIELD**

(57) It is an object of the present invention to provide a solution by providing a specific process that enables a dialkyl carbonate and a diol to be produced on an industrial scale of not less than 2 ton / hr and not less than 1.3 ton / hr respectively with high selectivity and high productivity stably for a prolonged period of time through a reactive distillation system of taking a cyclic carbonate and an aliphatic monohydric alcohol as starting materials, continuously feeding the starting materials into a continuous multi-stage distillation column in which a catalyst is present, and carrying out reaction and distillation simultaneously in the column. Although there have been many proposals regarding processes for the production of the dialkyl carbonate and the diol through the reactive distillation method, these have all been on a small scale and short operating time laboratory level, and there have been no disclosures whatsoever on a specific process or apparatus enabling mass production on an industrial scale. According to the present invention, there are provided a specific continuous multi-stage distillation column having a specified structure, and a production process using this continuous multi-stage distillation column, in which the dialkyl carbonate and the diol can be produced on an industrial scale of not less than 2 ton / hr and not less than 1.3 ton / hr respectively each with a selectivity of not less than 95%, preferably not less than 97%, more preferably not less than 99%, stably for not less than 1000 hours, preferably not less than 3000 hours, more preferably not less than 5000 hours.

**FIG.1**

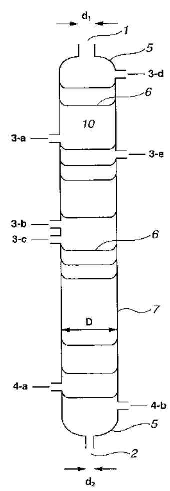


EP 1 961 723 A1

## Description

### Technical Field

The present invention relates to an industrial process for the production of a dialkyl carbonate and a diol with a high yield. More particularly, the present invention relates to a process for industrially producing large amounts of the dialkyl carbonate and the diol with a high yield stably for a prolonged period of time through a reactive distillation system of taking a cyclic carbonate and an aliphatic monohydric alcohol as starting materials, continuously feeding the starting materials into a continuous multi-stage distillation column in which a catalyst is present, and carrying out reaction and distillation simultaneously in the column.

### Background Art

Several processes for the production of a dialkyl carbonate and a diol from a reaction between a cyclic carbonate and an aliphatic monohydric alcohol have been proposed, but most of these proposals relate to a catalyst. As reaction systems, four systems have been proposed hitherto. These four reaction systems are used in a process for the production of dimethyl carbonate and ethylene glycol from ethylene carbonate and methanol, which is the most typical reaction example.

A first system is a completely batch reaction system in which ethylene carbonate, methanol and a catalyst are put into an autoclave, which is a batch reaction vessel, and reaction is carried out by holding for a predetermined reaction time under an applied temperature at a reaction temperature above the boiling point of methanol (see, for example, Patent Document 1: U.S. Patent No. 3642858, Patent Document 2: Japanese Patent Application Laid-Open No. S54-48715 (corresponding to U.S. Patent No. 4181676), Patent Document 5: Japanese Patent Application Laid-Open No.S54-63023, Patent Document 6: Japanese Patent Application Laid-Open No. S54-148726).

A second system is a system that uses an apparatus in which a distillation column is provided on top of a reaction vessel; ethylene carbonate, methanol and a catalyst are put into the reaction vessel, and reaction is made to proceed by heating to a predetermined temperature. With this system, to make up for methanol distilled off through azeotropy with the produced dimethyl carbonate, methanol is added to the reaction vessel continuously or in batches, but in any event with this system the reaction proceeds only in the reaction vessel, which is batch type, in which the catalyst, ethylene carbonate and methanol are present. The reaction is thus batch type, the reaction being carried out using a large excess of methanol under reflux taking a long time in a range of from 3 to over 20 hours (see, for example, Patent Document 3: Japanese Patent Application Laid-Open No. S51-122025 (corresponding to U.S. Patent No. 4062884), Patent Document 4: Japanese Patent Application Laid-Open No. S54-48716 (corresponding to U.S. Patent No. 4307032), Patent Document 11: U.S. Patent No. 3803201).

A third system is a continuous reaction system in which a mixed solution of ethylene carbonate and methanol is continuously fed into a tubular reactor maintained at a predetermined reaction temperature, and a reaction mixture containing ethylene carbonate and methanol which are unreacted, and dimethyl carbonate and ethylene glycol which are produced is continuously withdrawn in a liquid form from an outlet on the other side. Either of two processes is used depending on the form of the catalyst used. That is, there are a process in which a homogeneous catalyst is used, and is passed through the tubular reactor together with the mixed solution of ethylene carbonate and methanol, and then after the reaction the catalyst is separated out from the reaction mixture (see, for example, Patent Document 7: Japanese Patent Application Laid-Open No. 63-41432 (corresponding to U.S. Patent No. 4661609), Patent Document 10: U.S. Patent No. 4734518), and a process in which a heterogeneous catalyst fixed inside the tubular reactor is used (see, for example, Patent Document 8: Japanese Patent Application Laid-Open No. S63-238043, Patent Document 9: Japanese Patent Application Laid-Open No. S64-31737 (corresponding to U.S. Patent No. 4691041)). The reaction producing dimethyl carbonate and ethylene glycol through reaction between ethylene carbonate and methanol is an equilibrium reaction, and hence with this continuous flow reaction system using a tubular reactor, it is impossible to make the ethylene carbonate conversion higher than the equilibrium conversion determined by the composition ratio put in and the reaction temperature. For example, according to Example 1 in Patent Document 7 (Japanese Patent Application Laid-Open No. S63-41432 (corresponding to U.S. Patent No. 4661609)), for a flow reaction at 130°C using a starting material put in with a molar ratio of methanol / ethylene carbonate = 4 / 1, the ethylene carbonate conversion is 25%. This means that a large amount of unreacted ethylene carbonate and methanol remaining in the reaction mixture must be separated out, recovered, and recirculated back into the reactor, and in actual fact, with the process of Patent Document 9 (Japanese Patent Application Laid-Open No. S64-31737 (corresponding to U.S. Patent No. 4691041)), much equipment is used for such separation, purification, recovery, and recirculation.

A fourth system is a reactive distillation system first disclosed by the present inventors (see, for example, Patent Document 12: Japanese Patent Application Laid-Open No. H4-198141, Patent Document 13: Japanese Patent Application Laid-Open No. H4-230243, Patent Document 14: Japanese Patent Application Laid-Open No. H9-176061, Patent

Document 15: Japanese Patent Application Laid-Open No. H9-183744, Patent Document 16: Japanese Patent Application Laid-Open No. H9-194435, Patent Document 17: International Publication No. WO97/23445 (corresponding to European Patent No. 0889025, U.S. Patent No. 5847189), Patent Document 18: International Publication No. WO99/64382 (corresponding to European Patent No. 1086940, U.S. Patent No. 6346638), Patent Document 19: International Publication No. WO00/51954 (corresponding to European Patent No. 1174406, U.S. Patent No. 6479689), Patent Document 20: Japanese Patent Application Laid-Open No. 2002-308804, Patent Document 21: Japanese Patent Application Laid-Open No. 2004-131394), that is a continuous production process in which ethylene carbonate and methanol are each continuously fed into a multi-stage distillation column, and reaction is carried out in the presence of a catalyst in a plurality of stages in the distillation column, while the product dimethyl carbonate and ethylene glycol are separated off. Patent applications in which such a reactive distillation system is used have subsequently been filed by other companies (see, for example, Patent Document 22: Japanese Patent Application Laid-Open No. H5-213830 (corresponding to European Patent No. 0530615, U.S. Patent No. 5231212), Patent Document 23: Japanese Patent Application Laid-Open No. H6-9507 (corresponding to European Patent No. 0569812, U.S. Patent No. 5359118), Patent Document 24: Japanese Patent Application Laid-Open No. 2003-119168 (corresponding to International Publication No. WO03/006418), Patent Document 25: Japanese Patent Application Laid-Open No. 2003-300936, Patent Document 26: Japanese Patent Application Laid-Open No. 2003-342209).

In this way, the processes proposed hitherto for producing the dialkyl carbonate and the diol from the cyclic carbonate and the aliphatic monohydric alcohol are the four systems:

(1) a completely batch reaction system;
(2) a batch reaction system using a reaction vessel having a distillation column provided on top thereof;
(3) a flowing liquid reaction system using a tubular reactor; and
(4) a reactive distillation system.

However, there have been problems with these as follows.

In the case of (1) and (3), the upper limit of the cyclic carbonate conversion is determined by the composition put in and the temperature, and hence the reaction cannot be carried out to completion, and thus the conversion is low. Moreover, in the case of (2), to make the cyclic carbonate conversion high, the produced dialkyl carbonate must be distilled off using a very large amount of the aliphatic monohydric alcohol, and a long reaction time is required. In the case of (4), the reaction can be made to proceed with a higher conversion than with (1), (2) or (3). However, processes of (4) proposed hitherto have related to producing the dialkyl carbonate and the diol either in small amounts or for a short period of time, and have not related to carrying out the production on an industrial scale stably for a prolonged period of time. That is, these processes have not attained the object of producing a dialkyl carbonate continuously in a large amount (e.g. not less than 2 ton / hr) stably for a prolonged period of time (e.g. not less than 1000 hours, preferably not less than 3000 hours, more preferably not less than 5000 hours).

For example, the maximum values of the height (H: cm), diameter (D: cm), and number of stages (n) of the reactive distillation column, the amount produced P (kg / hr) of dimethyl carbonate, and the continuous production time T (hr) in examples disclosed for the production of dimethyl carbonate (DMC) and ethylene glycol (EG) from ethylene carbonate and methanol are as in Table 1.

Table 1

**TABLE 1**

| PATENT DOCUMENT | H: cm | D : cm | NO. STAGES : n | P : kg/hr | T : hr |
|---|---|---|---|---|---|
| 12 | 100 | 2 | 30 | 0.106 | 400 |
| 15 | 160 | 5 | 40 | 0.427 | NOTE 5 |
| 16 | 160 | 5 | 40 | 0.473 | NOTE 5 |
| 18 | 200 | 4 | PACKING COLUMN (Dixon) | 0.932 | NOTE 5 |
| 19 | NOTE 1 | 5 | 60 | 0.275 | NOTE 5 |
| 20 | NOTE 1 | 5 | 60 | 0.258 | NOTE 5 |
| 21 | NOTE 1 | 5 | 60 | 0.258 | NOTE 5 |
| 22 | 250 | 3 | PACKING COLUMN (Raschig) | 0.392 | NOTE 5 |
| 23 | NOTE 2 | NOTE 2 | NOTE 2 | 0.532 | NOTE 5 |
| 24 | NOTE 3 | NOTE 3 | 42 | NOTE 4 | NOTE 5 |

(continued)

| PATENT DOCUMENT | H: cm | D : cm | NO. STAGES : n | P : kg/hr | T : hr |
|---|---|---|---|---|---|
| 25 | NOTE 3 | NOTE 3 | 30 | 3750 | NOTE 5 |
| 26 | 200 | 15 | PACKING COLUMN (BX) | 0.313 | NOTE 5 |

NOTE 1 : OLDERSHAW DISTILLATION COLUMN.
NOTE 2 : NO DESCRIPTION WHATSOEVER DEFINING DISTILLATION COLUMN.
NOTE 3 : ONLY DESCRIPTION DEFINING DISTILLATION COLUMN IS NUMBER OF STAGES.
NOTE 4 : NO DESCRIPTION WHATSOEVER OF PRODUCED AMOUNT.
NOTE 5 : NO DESCRIPTION WHATSOEVER REGARDING STABLE PRODUCTION FOR PROLONGED PERIOD OF TIME.

In Patent Document 25 (Japanese Patent Application Laid-Open No. 2003-300936) (paragraph 0060), it is stated that "The present example uses the same process flow as for the preferred mode shown in FIG. 1 described above, and was carried out with the object of operating a commercial scale apparatus for producing dimethyl carbonate and ethylene glycol through transesterification by a catalytic conversion reaction between ethylene carbonate and methanol. Note that the following numerical values in the present example can be adequately used in the operation of an actual apparatus", and as that example it is stated that 3750 kg / hr of dimethyl carbonate was specifically produced. The scale described in that example corresponds to an annual production of 30,000 tons or more, and hence this implies that at the time of the filing of the patent application for Patent Document 25 (Japanese Patent Application Laid-Open No. 2003-300936) (April 9, 2002), operation of the world's first large scale commercial plant using this process had been carried out. However, even at the time of filing the present application, there is not the above fact at all. Moreover, in the example of Patent Document 25 (Japanese Patent Application Laid-Open No. 2003-300936), exactly the same value as the theoretically calculated value is stated for the produced amount of dimethyl carbonate, but the yield for ethylene glycol is approximately 85.6%, and the selectivity is approximately 88.4%, and hence it cannot really be said that a high yield and high selectivity have been attained. In particular, the low selectivity indicates that this process has a fatal drawback as an industrial production process. (Note also that Patent Document 25 (Japanese Patent Application Laid-Open No. 2003-300936) was deemed to have been withdrawn on July 26, 2005 due to examination not having been requested).

With the reactive distillation method, there are very many causes of fluctuation such as composition variation due to reaction and composition variation due to distillation in the distillation column, and temperature variation and pressure variation in the column, and hence continuing stable operation for a prolonged period of time is accompanied by many difficulties, and in particular these difficulties are further increased in the case of handling large amounts. To continue mass production of the dialkyl carbonate and the diol using the reactive distillation method stably for a prolonged period of time while maintaining high yields and high selectivities for the dialkyl carbonate and the diol, the reactive distillation apparatus must be cleverly devised. However, the only description of continuous stable production for a prolonged period of time with the reactive distillation method proposed hitherto has been the 200 to 400 hours in Patent Document 12 (Japanese Patent Application Laid-Open No. H4-198141) and Patent Document 13 (Japanese Patent Application Laid-Open No. H4-230243).

Disclosure of Invention

Problems to be Solved by the Invention

It is an object of the present invention to provide, for the case of producing a dialkyl carbonate and a diol industrially in large amounts (e.g. not less than 2 ton / hr for the dialkyl carbonate, and not less than 1.3 ton / hr for the diol) through a reactive distillation system of taking a cyclic carbonate and an aliphatic monohydric alcohol as starting materials, continuously feeding the starting materials into a continuous multi-stage distillation column in which a homogeneous catalyst is present, and carrying out reaction and distillation simultaneously in the column, a specific process in which the dialkyl carbonate and the diol can be produced with high selectivity and high productivity stably for a prolonged period of time (e.g. not less than 1000 hours, preferably not less than 3000 hours, more preferably not less than 5000 hours) with a high yield.

Means for Solving the Problems

Since the present inventors first disclosed a process for continuously producing the dialkyl carbonate and the

diol using the continuous multi-stage distillation column, there have been many proposals regarding improving this process. However, these have been on a small scale and short operating time laboratory level, and there have been no disclosures whatsoever on a specific process or apparatus enabling mass production on an industrial scale stably for a prolonged period of time based on findings obtained through actual implementation. The present inventors have thus carried out studies aimed at discovering a specific process enabling the dialkyl carbonate and the diol to be produced on an industrial scale of, for example, not less than 2 ton / hr for the dialkyl carbonate and not less than 1.3 ton / hr for the diol stably with a high yield for a prolonged period of time with high selectivity and high productivity. As a result, the present inventors have arrived at the present invention.

That is, according to the first aspect of the present invention, there are provided:

1. a process for producing a dialkyl carbonate and a diol industrially with a high yield in which the dialkyl carbonate and the diol are continuously produced through a reactive distillation system of taking a cyclic carbonate and an aliphatic monohydric alcohol as starting materials, comprising the steps of:

continuously feeding the starting materials into a continuous multi-stage distillation column in which a homogeneous catalyst is present;
carrying out reaction and distillation simultaneously in the column;
continuously withdrawing a low boiling point reaction mixture containing the produced dialkyl carbonate from an upper portion of said column in a gaseous form; and
continuously withdrawing a high boiling point reaction mixture containing the diol from a lower portion of the column in a liquid form,

wherein:

(a) said continuous multi-stage distillation column comprises a tray column type distillation column having a cylindrical trunk portion having a length L (cm) and an inside diameter D (cm) and having a structure having an internal with a number of stages n, said internal being a tray having a plurality of holes, and further having a gas outlet having an inside diameter $d_1$ (cm) at the top of the column or in the upper portion of the column near to the top, a liquid outlet having an inside diameter $d_2$ (cm) at the bottom of the column or in the lower portion of the column near to the bottom, at least one first inlet provided in the upper portion and / or a middle portion of the column below the gas outlet, and at least one second inlet provided in the middle portion and / or the lower portion of the column above the liquid outlet, wherein:

(1) the length L (cm) satisfies the formula (1);

$$2100 \leq L \leq 8000 \qquad (1),$$

(2) the inside diameter D (cm) of the column satisfies the formula (2);

$$180 \leq D \leq 2000 \qquad (2),$$

(3) a ratio of the length L (cm) to the inside diameter D (cm) of the column satisfies the formula (3);

$$4 \leq L / D \leq 40 \qquad (3),$$

(4) the number of stages n satisfies the formula (4);

$$10 \leq n \leq 120 \qquad (4),$$

(5) a ratio of the inside diameter D (cm) of the column to the inside diameter $d_1$ (cm) of the gas outlet

satisfies the formula (5);

$$3 \leq D / d_1 \leq 20 \qquad (5),$$

and

(6) a ratio of the inside diameter D (cm) of the column to the inside diameter $d_2$ (cm) of the liquid outlet satisfies the formula (6);

$$5 \leq D / d_2 \leq 30 \qquad (6);$$

(b) the starting material cyclic carbonate is continuously introduced into said continuous multi-stage distillation column from at least one said first inlet provided between the $3^{rd}$ stage from the top of said continuous multi-stage distillation column and the $(n/3)^{th}$ stage from the top of said continuous multi-stage distillation column;
(c) the starting material aliphatic monohydric alcohol is continuously introduced into said continuous multi-stage distillation column from at least one said second inlet provided between the $(n/3)^{th}$ stage from the top of said continuous multi-stage distillation column and the $(2n/3)^{th}$ stage from the top of said continuous multi-stage distillation column;
(d) an aperture ratio of the tray installed not lower than a stage where the cyclic carbonate is introduced is in a range of from 2.5 to 10%;
(e) an aperture ratio of the tray installed not higher than the stage where the cyclic carbonate is introduced and not lower than a stage where the aliphatic monohydric alcohol is introduced is in a range of from 2 to 8%; and
(f) an aperture ratio of the tray installed not higher than the stage where the aliphatic monohydric alcohol is introduced is in a range of from 1.5 to 5%,

2. the process according to item 1, wherein a produced amount of the dialkyl carbonate is not less than 2 ton / hr,
3. the process according to item 1 or 2, wherein the produced amount of the diol is not less than 1.3 ton / hr,
4. the process according to any one of items 1 to 3, wherein said $d_1$ and said $d_2$ satisfy the formula (7);

$$1 \leq d_1 / d_2 \leq 5 \qquad (7),$$

5. the process according to any one of items 1 to 4, wherein L, D, L / D, n, D / $d_1$, and D / $d_2$ for said continuous multi-stage distillation column satisfy respectively $2300 \leq L \leq 6000$, $200 \leq D \leq 1000$, $5 \leq L/D \leq 30$, $30 \leq n \leq 100$, $4 \leq D/d_1 \leq 15$, and $7 \leq D/d_2 \leq 25$,
6. the process according to any one of items 1 to 5, wherein L, D, L / D, n, D / $d_1$, and D / $d_2$ for said continuous multi-stage distillation column satisfy respectively $2500 \leq L \leq 5000$, $210 \leq D \leq 800$, $7 \leq L / D \leq 20$, $40 \leq n \leq 90$, $5 \leq D / d_1 \leq 13$, and $9 \leq D / d_2 \leq 20$,
7. the process according to any one of items 1 to 6, wherein the aperture ratio of the tray installed not lower than said stage where the cyclic carbonate is introduced is in a range of from 3 to 8%, the aperture ratio of the tray installed not higher than said stage where the cyclic carbonate is introduced and not lower than said stage where the aliphatic monohydric alcohol is introduced is in a range of from 2.5 to 7%, and the aperture ratio of the tray installed not higher than said stage where the aliphatic monohydric alcohol is introduced is in a range of from 1.8 to 4.5%,
8. the process according to any one of items 1 to 7, wherein the aperture ratio of the tray installed not lower than the stage where the cyclic carbonate is introduced is in a range of from 3.5 to 6%, the aperture ratio of the tray installed not higher than the stage where the cyclic carbonate is introduced and not lower than the stage where the aliphatic monohydric alcohol is introduced is in a range of from 3 to 5.5%, and the aperture ratio of the tray installed not higher than the stage where the aliphatic monohydric alcohol is introduced is in a range of from 2 to 4%,
9. the process according to any one of items 1 to 8, wherein said tray is a sieve tray having a sieve portion and a downcomer portion,
10. the process according to item 9, wherein said sieve trays has 100 to 1000 holes / $m^2$ in said sieve portion thereof,
11. the process according to item 9 or 10, wherein a cross-sectional area per hole of said sieve trays is in a range of from 0.5 to 5 $cm^2$.

In addition, according to the second aspect of the present invention, there are provided:
12. a continuous multi-stage distillation column for carrying out transesterification between a cyclic carbonate and an aliphatic monohydric alcohol and distillation, wherein:

(a) the continuous multi-stage distillation column comprises:

a cylindrical trunk portion having a length L (cm) and an inside diameter D (cm);
a tray having number of stages n provided inside the trunk portion;
a gas outlet having an inside diameter $d_1$ (cm) provided at a top of the column or in an upper portion of the column near to the top;
a liquid outlet having an inside diameter $d_2$ (cm) provided at a bottom of the column or in a lower portion of the column near to the bottom;
at least one first inlet provided in the upper portion and / or a middle portion of the column below said gas outlet; and
at least one second inlet provided in the middle portion and / or the lower portion of the column above said liquid outlet;

wherein:

(1) the length L (cm) satisfies the formula (1);

$$2100 \leq L \leq 8000 \qquad (1),$$

(2) the inside diameter D (cm) of the column satisfies the formula (2);

$$180 \leq D \leq 2000 \qquad (2),$$

(3) a ratio of the length L (cm) to the inside diameter D (cm) of the column satisfies the formula (3);

$$4 \leq L / D \leq 40 \qquad (3),$$

(4) the number of stages n satisfies the formula (4);

$$10 \leq n \leq 120 \qquad (4),$$

(5) a ratio of the inside diameter D (cm) of the column to the inside diameter $d_1$ (cm) of the gas outlet satisfies the formula (5);

$$3 \leq D / d_1 \leq 20 \qquad (5),$$

and
(6) a ratio of the inside diameter D (cm) of the column to the inside diameter $d_2$ (cm) of the liquid outlet satisfies the formula (6),

$$5 \leq D / d_2 \leq 30 \qquad (6);$$

(b) the starting material cyclic carbonate is continuously introduced into said continuous multi-stage distillation column from at least one said first inlet provided between the 3rd stage from the top of said continuous multi-stage distillation column and the $(n/3)^{th}$ stage from the top of said continuous multi-stage distillation column;
(c) the starting material aliphatic monohydric alcohol is continuously introduced into said continuous multi-stage distillation column from at least one said second inlet provided between the $(n/3)^{th}$ stage from the top of said continuous multi-stage distillation column and the $(2n/3)^{th}$ stage from the top of said continuous multi-stage distillation column;
(d) an aperture ratio of the tray installed not lower than a stage where the cyclic carbonate is introduced is in a range of from 2.5 to 10%;
(e) an aperture ratio of the tray installed not higher than the stage where the cyclic carbonate is introduced and not lower than a stage where the aliphatic monohydric alcohol is introduced is in a range of from 2 to 8%; and
(f) an aperture ratio of the tray installed not higher than the stage where the aliphatic monohydric alcohol is introduced is in a range of from 1.5 to 5%,

13. the continuous multi-stage distillation column according to item 12, wherein said $d_1$ and said $d_2$ satisfy the formula (7);

$$1 \leq d_1 / d_2 \leq 5 \qquad (7),$$

14. the continuous multi-stage distillation column according to item 12 or 13, wherein L, D, L / D, n, D / $d_1$, and D / $d_2$ for said continuous multi-stage distillation column satisfy respectively $2300 \leq L \leq 6000$, $200 \leq D \leq 1000$, $5 \leq L / D \leq 30$, $30 \leq n \leq 100$, $4 \leq D / d_1 \leq 15$, and $7 \leq D / d_2 \leq 25$,
15. the continuous multi-stage distillation column according to any one of items 12 to 14, wherein L, D, L / D, n, D / $d_1$, and D / $d_2$ for said continuous multi-stage distillation column satisfy respectively $2500 \leq L \leq 5000$, $210 \leq D \leq 800$, $7 \leq L / D \leq 20$, $40 \leq n \leq 90$, $5 \leq D / d_1 \leq 13$, and $9 \leq D / d_2 \leq 20$,
16. the continuous multi-stage distillation column according to any one of items 12 to 15, wherein the aperture ratio of the tray installed not lower than said stage where the cyclic carbonate is introduced is in a range of from 3 to 8%, the aperture ratio of the tray installed not higher than said stage where the cyclic carbonate is introduced and not lower than said stage where the aliphatic monohydric alcohol is introduced is in a range of from 2.5 to 7%, and the aperture ratio of the tray installed not higher than said stage where the aliphatic monohydric alcohol is introduced is in a range of from 1.8 to 4.5%,
17. the continuous multi-stage distillation column according to any one of items 12 to 16, wherein the aperture ratio of the tray installed not lower than said stage where the cyclic carbonate is introduced is in a range of from 3.5 to 6%, the aperture ratio of the tray installed not higher than said stage where the cyclic carbonate is introduced and not lower than said stage where the aliphatic monohydric alcohol is introduced is in a range of from 3 to 5.5%, and the aperture ratio of the trays installed not higher than said stage where the aliphatic monohydric alcohol is introduced is in a range of from 2 to 4%,
18. the continuous multi-stage distillation column according to any one of items 12 to 17, wherein said tray is a sieve tray having a sieve portion and a downcomer portion,
19. the continuous multi-stage distillation column according to item 18, wherein said sieve tray has 100 to 1000 holes / $m^2$ in said sieve portion thereof,
20. the continuous multi-stage distillation column according to item 18 or 19, wherein a cross-sectional area per hole of said sieve tray is in a range of from 0.5 to 5 $cm^2$.

Advantageous Effects of Invention

It has been discovered that by implementing the present invention, the dialkyl carbonate and the diol can be produced each with a high yield and a high selectivity of not less than 95%, preferably not less than 97%, more preferably not less than 99%, on an industrial scale of not less than 2 ton /hr, preferably not less than 3 ton / hr, more preferably not less than 4 ton / hr, for the dialkyl carbonate, and not less than 1.3 ton / hr, preferably not less than 1.95 ton / hr, more preferably not less than 2.6 ton / hr, for the diol, stably for a prolonged period of time of not less than 1000 hours, preferably not less than 3000 hours, more preferably not less than 5000 hours, from a cyclic carbonate and an aliphatic monohydric alcohol.

DESCRIPTION OF DRAWINGS

FIG. 1 is an example of schematic drawing of the continuous multi-stage distillation column for carrying out the present invention, the distillation column having number of stages n of trays as the internals (in FIG. 1, a tray is shown schematically) provided inside a trunk portion thereof.

Description of Reference Numerals:

1: gas outlet, 2: liquid outlet, 3-a to 3-e: inlet, 4-a to 4-b: inlet, 5: end plate, 6: internal, 7: trunk portion, 10: continuous multi-stage distillation column, L: length (cm) of trunk portion, D: inside diameter (cm) trunk portion, $d_1$: inside diameter (cm) of gas outlet, $d_2$ : inside diameter (cm) of liquid outlet.

Best Mode for Carrying Out the Invention

Following is a detailed description of the present invention.

The reaction of the present invention is a reversible equilibrium transesterification reaction represented by following formula in which a dialkyl carbonate (C) and a diol (D) are produced from a cyclic carbonate (A) and an aliphatic monohydric alcohol (B).

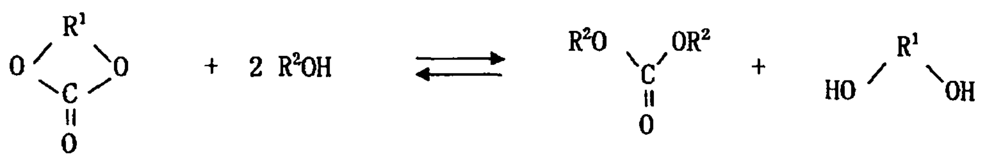

(A) (B) (C) (D)

wherein $R^1$ represents a bivalent group $-(CH_2)_m-$ (m is an integer from 2 to 6), one or more of the hydrogens thereof being optionally substituted with an alkyl group or aryl group having 1 to 10 carbon atoms. Moreover, $R^2$ represents a monovalent aliphatic group having 1 to 12 carbon atoms, one or more of the hydrogens thereof being optionally substituted with an alkyl group or aryl group having 1 to 10 carbon atoms.

The cyclic carbonate used as a starting material in the present invention is a compound represented by (A) in the above formula. For example, an alkylene carbonate such as ethylene carbonate or propylene carbonate, or 1,3-dioxacyclohexa-2-one, 1,3-dioxacyclohepta-2-one, or the like can be preferably used, ethylene carbonate or propylene carbonate being more preferably used due to ease of procurement and so on, and ethylene carbonate being particularly preferably used.

Moreover, the aliphatic monohydric alcohol used as the other starting material is a compound represented by (B) in the above formula. An aliphatic monohydric alcohol having a lower boiling point than that of the produced diol is used. Although possibly varying depending on the type of the cyclic carbonate used, examples of aliphatic monohydric alcohol include methanol, ethanol, propanol (isomers), allyl alcohol, butanol (isomers), 3-buten-1-ol, amyl alcohol (isomers), hexyl alcohol (isomers), heptyl alcohol (isomers), octyl alcohol (isomers), nonyl alcohol (isomers), decyl alcohol (isomers), undecyl alcohol (isomers), dodecyl alcohol (isomers), cyclopentanol, cyclohexanol, cycloheptanol, cyclooctanol, methylcyclopentanol (isomers), ethylcyclopentanol (isomers), methylcyclohexanol (isomers), ethylcyclohexanol (isomers), dimethylcyclohexanol (isomers), diethylcyclohexanol (isomers), phenylcyclohexanol (isomers), benzyl alcohol, phenethyl alcohol (isomers), phenylpropanol (isomers), and so on. Furthermore, these aliphatic monohydric alcohols may be substituted with substituents such as halogens, lower alkoxy groups, cyano groups, alkoxycarbonyl groups, aryloxycarbonyl groups, acyloxy groups, and nitro groups.

Of such aliphatic monohydric alcohols, ones preferably used are alcohols having 1 to 6 carbon atoms, more preferably alcohols having 1 to 4 carbon atoms, i.e. methanol, ethanol, propanol (isomers), and butanol (isomers). In the case of using ethylene carbonate or propylene carbonate as the cyclic carbonate, preferable aliphatic monohydric alcohols are methanol and ethanol, methanol being particularly preferable.

In the process of the present invention, a homogeneous catalyst is made to be present in the reactive distillation

column. The method of making the homogeneous catalyst be present may be any method, but it is preferable to feed the catalyst into the reactive distillation column continuously so as to make the catalyst be present in a liquid phase in the reactive distillation column.

In the case that the homogeneous catalyst is continuously fed into the reactive distillation column, the homogeneous catalyst may be fed in together with the cyclic carbonate and/or the aliphatic monohydric alcohol, or may be fed in at a different position to the starting materials. The reaction actually proceeds in the distillation column in a region below the position at which the catalyst is fed in, and hence it is preferable to feed the catalyst into a region between the top of the column and the position(s) at which the starting materials are fed in. The catalyst must be present in at least 5 stages, preferably at least 7 stages, more preferably at least 10 stages.

As the catalyst used in the present invention, any of various catalysts known from hitherto can be used. Examples of the catalyst include:

alkali metals and alkaline earth metals such as lithium, sodium, potassium, rubidium, cesium, magnesium, calcium, strontium, and barium;
basic compounds of alkali metals and alkaline earth metals such as hydrides, hydroxides, alkoxides, aryloxides, and amides;
basic compounds of alkali metals and alkaline earth metals such as carbonates, bicarbonates, and organic acid salts;
tertiary amines such as triethylamine, tributylamine, trihexylamine, and benzyldiethylamine;
nitrogen-containing heteroaromatic compounds such as N-alkylpyrroles, N-alkylindoles, oxazoles, N-alkylimidazoles, N-alkylpyrazoles, oxadiazoles, pyridine, alkylpyridines, quinoline, alkylquinolines, isoquinoline, alkylisoquinolines, acridine, alkylacridines, phenanthroline, alkylphenanthrolines, pyrimidine, alkylpyrimidines, pyrazine, alkylpyrazines, triazines, and alkyltriazines;
cyclic amidines such as diazobicycloundecene (DBU) and diazobicyclononene (DBN);
thallium compounds such as thallium oxide, thallium halides, thallium hydroxide, thallium carbonate, thallium nitrate, thallium sulfate, and thallium organic acid salts;
tin compounds such as tributylmethoxytin, tributylethoxytin, dibutyldimethoxytin, diethyldiethoxytin, dibutyldiethoxytin, dibutylphenoxytin, diphenylmethoxytin, dibutyltin acetate, tributyltin chloride, and tin 2-ethylhexanoate;
zinc compounds such as dimethoxyzinc, diethoxyzinc, ethylenedioxyzinc, and dibutoxyzinc; aluminum compounds such as aluminum trimethoxide, aluminum triisopropoxide, and aluminum tributoxide;
titanium compounds such as tetramethoxytitanium, tetraethoxytitanium, tetrabutoxytitanium, dichlorodimethoxytitanium, tetraisopropoxytitanium, titanium acetate, and titanium acetylacetonate;
phosphorus compounds such as trimethylphosphine, triethylphosphine, tributylphosphine, triphenylphosphine, tributylmethylphosphonium halides, trioctylbutylphosphonium halides, and triphenylmethylphosphonium halides;
zirconium compounds such as zirconium halides, zirconium acetylacetonate, zirconium alkoxides, and zirconium acetate; and
lead and lead-containing compounds, for example lead oxides such as $PbO$, $PbO_2$, and $Pb_3O_4$; lead sulfides such as $PbS$, $Pb_2S_3$, and $PbS_2$;
lead hydroxides such as $Pb(OH)_2$, $Pb_3O_2(OH)_2$, $Pb_2[PbO_2(OH)_2]$, and $Pb_2O(OH)_2$;
plumbites such as $Na_2PbO_2$, $K_2PbO_2$, $NaHPbO_2$, and $KHPbO_2$;
plumbates such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$;
lead carbonates and basic salts thereof such as $PbCO_3$ and $2PbCO_3 \cdot Pb(OH)_2$;
alkoxylead compounds and aryloxylead compounds such as $Pb(OCH_3)_2$, $(CH_3O)Pb(OPh)$, and $Pb(OPh)_2$;
lead salts of organic acids, and carbonates and basic salts thereof, such as $Pb(OCOCH_3)_2$, $Pb(OCOCH_3)_4$, and $Pb(OCOCH_3)_2 \cdot PbO \cdot 3H_2O$;
organolead compounds such as $Bu_4Pb$, $Ph_4Pb$, $Bu_3PbCl$, $Ph_3PbBr$, $Ph_3Pb$ (or $Ph_6Pb_2$), $Bu_3PbOH$, and $Ph_2PbO$ (wherein Bu represents a butyl group, and Ph represents a phenyl group);
lead alloys such as Pb-Na, Pb-Ca, Pb-Ba, Pb-Sn, and Pb-Sb;
lead minerals such as galena and zinc blende; and
hydrates of such lead compounds.

In the case that the compound used dissolves in a starting material of the reaction, the reaction mixture, a reaction by-product or the like, the compound can be used as is as the homogeneous catalyst. Alternatively, it is also preferable to use, as the homogeneous catalyst, a mixture obtained by dissolving the above compound in the starting material of the reaction, the reaction mixture, the reaction by-product or the like in advance, or by reacting to bring about dissolution.

The amount of the catalyst used in the present invention varies depending on the type of the catalyst used, but is generally in a range of from 0.0001 to 50 % by weight, preferably from 0.005 to 20 % by weight, more preferably from 0.01 to 10 % by weight, as a proportion of the total weight of the cyclic carbonate and the aliphatic monohydric alcohol

fed in as the starting materials.

When continuously feeding the cyclic carbonate into the continuous multi-stage distillation column constituting the reactive distillation column in the present invention, it is important for the cyclic carbonate to be fed into a specified stage. Specifically, the starting material cyclic carbonate must be continuously introduced into the continuous multi-stage distillation column from at least one inlet provided between the 3$^{rd}$ stage from the top of the continuous multi-stage distillation column and the (n/3)$^{th}$ stage from the top of the continuous multi-stage distillation column. This is important to ensure that high boiling point compounds such as the cyclic carbonate and the diol are not contained in a column top component in the stages above the cyclic carbonate inlet(s). For this reason, there are preferably not less than 3 stages, more preferably 4 to 10 stages, yet more preferably 5 to 8 stages, above the cyclic carbonate inlet(s).

A cyclic carbonate preferably used in the present invention is one not containing a halogen that has been produced through reaction between, for example, an alkylene oxide such as ethylene oxide, propylene oxide or styrene oxide and carbon dioxide. A cyclic carbonate containing small amounts of such raw material compounds, the diol, and so on may thus be used as the starting material in the present invention.

In the present invention, the starting material aliphatic monohydric alcohol may be a high-purity aliphatic monohydric alcohol, or may contain other compounds. For example, it is preferable to use an aliphatic monohydric alcohol containing in a range of from 1 to 15 % by weight of the dialkyl carbonate based on the total mass of the aliphatic monohydric alcohol and the dialkyl carbonate. It is more preferable to use an aliphatic monohydric alcohol having this dialkyl carbonate content in a range of from 1.5 to 12 % by weight, yet more preferably from 2 to 10 % by weight.

When carrying out the present reaction industrially, besides fresh cyclic carbonate and/or aliphatic monohydric alcohol newly introduced into the reaction system, material having the cyclic carbonate and/or the aliphatic monohydric alcohol as a main component thereof recovered from this process and/or another process can also be preferably used for the starting materials. It is an excellent characteristic feature of the present invention that this is possible. An example of another process is a process in which a diaryl carbonate is produced from the dialkyl carbonate and the aromatic monbhydroxy compound, the aliphatic monohydric alcohol being by-produced in this process and recovered. The recovered by-produced aliphatic monohydric alcohol generally contains the dialkyl carbonate, but it has been discovered that in the case that the content of the dialkyl carbonate is in a range as above, excellent effects of the present invention can be achieved. The recovered by-produced aliphatic monohydric alcohol may further contain the aromatic monohydroxy compound, an alkyl aryl ether, small amounts of an alkyl aryl carbonate and the diaryl carbonate, and so on. The by-produced aliphatic monohydric alcohol may be used as is as a starting material in the present invention, or may be used as the starting material after the amount of contained material having a higher boiling point than that of the aliphatic monohydric alcohol has been reduced through distillation or the like.

When continuously feeding the aliphatic monohydric alcohol into the continuous multi-stage distillation column constituting the reactive distillation column in the present invention, it is important for the aliphatic monohydric alcohol to be fed into a specified stage. Specifically, in the present invention, the starting material aliphatic monohydric alcohol must be continuously introduced into the continuous multi-stage distillation column from at least one inlet provided between the (n/3)$^{th}$ stage from the top of the continuous multi-stage distillation column and the (2n/3)$^{th}$ stage from the top of the continuous multi-stage distillation column. It has been discovered that, because the aliphatic monohydric alcohol used as a starting material in the present invention generally contains a specified amount of the dialkyl carbonate, excellent effects of the present invention can be achieved by making the aliphatic monohydric alcohol inlet(s) be in a stage within this specified range. More preferably, the aliphatic monohydric alcohol is continuously introduced into the continuous multi-stage distillation column from at least one inlet provided between the (2n/5)$^{th}$ stage from the top of the continuous multi-stage distillation column and the (3n/5)$^{th}$ stage from the top of the continuous multi-stage distillation column.

The starting materials are fed continuously into the distillation column in a liquid form, in a gaseous form, or as a mixture of a liquid and a gas. Other than feeding the starting materials into the distillation column in this way, it is also preferable to additionally feed in a gaseous starting material intermittently or continuously from a middle portion and/or a lower portion of the distillation column. Moreover, another preferable method is one in which the cyclic carbonate is continuously fed in a liquid form or a gas / liquid mixed form into a stage of the distillation column above the stages in which the catalyst is present, and the aliphatic monohydric alcohol is continuously fed into the distillation column in a gaseous form and/or a liquid form from at least one inlet provided in a stage of the distillation column as described above. The starting materials are preferably made to contact the catalyst in a region of at least 5 stages, preferably at least 7 stages, more preferably at least 10 stages, of the distillation column.

In the present invention, a ratio between the amounts of the cyclic carbonate and the aliphatic monohydric alcohol fed into the reactive distillation column varies according to the type and amount of the transesterification catalyst and the reaction conditions, but the molar ratio of the aliphatic monohydric alcohol to the cyclic carbonate fed in is generally in a range of from 0.01 to 1000 times. To increase the cyclic carbonate conversion, it is preferable to feed in the aliphatic monohydric alcohol in an excess of at least 2 times the number of mols of the cyclic carbonate, but if the amount of the aliphatic monohydric alcohol used is too great, then it is necessary to make the apparatus larger. For

such reasons, the molar ratio of the aliphatic monohydric alcohol to the cyclic carbonate is preferably in a range of from 2 to 20, more preferably from 3 to 15, yet more preferably from 5 to 12. Furthermore, if much unreacted cyclic carbonate remains, then the unreacted cyclic carbonate may react with the product diol to by-produce oligomers such as a dimer or a trimer, and hence in industrial implementation, it is preferable to reduce the amount of unreacted cyclic carbonate remaining as much as possible. In the process of the present invention, even if the above molar ratio is not more than 10, the cyclic carbonate conversion can be made to be not less than 98%, preferably not less than 99%, more preferably not less than 99.9%. This is another characteristic feature of the present invention.

In the present invention, preferably not less than 2 ton / hr of the dialkyl carbonate is continuously produced; the minimum amount of the cyclic carbonate continuously fed in to achieve this is generally 2.2 P ton / hr, preferably 2.1 P ton / hr, more preferably 2.0 P ton / hr, based on the amount P (ton / hr) of the dialkyl carbonate to be produced. In a yet more preferable case, this amount can be made to be less than 1.9 P ton / hr.

FIG. 1 shows an example of schematic drawing of the continuous multi-stage distillation column for carrying out the production process according to the present invention. Here, the continuous multi-stage distillation column 10 used in the present invention comprises a tray column type distillation column which has a structure having end plates 5 respectively above and below a cylindrical trunk portion 7 having a length L (cm) and an inside diameter D (cm), and having thereinside an internal with a number of stages n, said internals being trays 6 each having a plurality of holes, and which further has a gas outlet 1 having an inside diameter $d_1$ (cm) at the top of the column or in an upper portion of the column near to the top, a liquid outlet 2 having an inside diameter $d_2$ (cm) at the bottom of the column or in a lower portion of the column near to the bottom, at least one first inlet 3 (a, e) provided below the gas outlet 1 between the 3$^{rd}$ stage from the top of the continuous multi-stage distillation column and the (n/3)$^{th}$ stage from the top of the continuous multi-stage distillation column, and at least one second inlet 3 (b, c) and 4 (a, b) provided above the liquid outlet 2 between the (n/3)$^{th}$ stage from the top of the continuous multi-stage distillation column and the (2n/3)$^{th}$ stage from the top of the continuous multi-stage distillation column, and moreover must be made to satisfy various conditions so as to be able to carry out not only distillation but also reaction at the same time so as to be able to produce preferably not less than 2 ton / hr of the dialkyl carbonate and/or preferably not less than 1.3 ton / hr of the diol stably for a prolonged period of time. Note that FIG. 1 is merely one embodiment of the continuous multi-stage distillation column according to the present invention, and hence the arrangement of the tray stages is not limited to that shown in FIG. 1.

The continuous multi-stage distillation column according to the present invention satisfies not only conditions from the perspective of the distillation function, but rather these conditions are combined with conditions required to make the reaction proceed stably with a high conversion and high selectivity, specifically the following must be satisfied:

(a) in the continuous multi-stage distillation column,

(1) the length L (cm) satisfies the formula (1);

$$2100 \leq L \leq 8000 \qquad (1),$$

(2) the inside diameter D (cm) of the column satisfies the formula (2);

$$180 \leq D \leq 2000 \qquad (2),$$

(3) a ratio of the length L (cm) to the inside diameter D (cm) of the column satisfies the formula (3);

$$4 \leq L / D \leq 40 \qquad (3),$$

(4) the number of stages n satisfies the formula (4);

$$10 \leq n \leq 120 \qquad (4),$$

(5) a ratio of the inside diameter D (cm) of the column to the inside diameter $d_1$ (cm) of the gas outlet satisfies

the formula (5);

$$3 \leq D / d_1 \leq 20 \qquad (5),$$

and
(6) a ratio of the inside diameter D (cm) of the column to the inside diameter $d_2$ (cm) of the liquid outlet satisfies formula (6);

$$5 \leq D / d_2 \leq 30 \qquad (6),$$

(b) the starting material cyclic carbonate is continuously introduced into the continuous multi-stage distillation column from at least one said first inlet provided between the 3rd stage from the top of the continuous multi-stage distillation column and the $(n/3)^{th}$ stage from the top of the continuous multi-stage distillation column;
(c) the starting material aliphatic monohydric alcohol is continuously introduced into the continuous multi-stage distillation column from at least one said second inlet provided between the $(n/3)^{th}$ stage from the top of the continuous multi-stage distillation column and the $(2n/3)^{th}$ stage from the top of the continuous multi-stage distillation column;
(d) an aperture ratio of a tray installed not lower than a stage where the cyclic carbonate is introduced is in a range of from 2.5 to 10%;
(e) an aperture ratio of a tray installed not higher than the stage where the cyclic carbonate is introduced and not lower than a stage where the aliphatic monohydric alcohol is introduced is in a range of from 2 to 8%; and
(f) an aperture ratio of a tray installed not higher than the stage where the aliphatic monohydric alcohol is introduced is in a range of from 1.5 to 5%.

Note that the term "the top of the column or the upper portion of the column near to the top" used in the present invention means the portion from the top of the column downward as far as approximately 0.25 L, and the term "the bottom of the column or the lower portion of the column near to the bottom" means the portion from the bottom of the column upward as far as approximately 0.25 L. Here, "L" is defined above.

It has been discovered that by using the specified continuous multi-stage distillation column that simultaneously satisfies the formulae (1) to (6), and in which the starting material cyclic carbonate and aliphatic monohydric alcohol inlets are respectively within the specified ranges described above, and the aperture ratios of the tray satisfy the ranges described above, the dialkyl carbonate and the diol can be produced on an industrial scale of preferably not less than 2 ton / hr of the dialkyl carbonate and/or preferably not less than 1.3 ton / hr of the diol with a high conversion, high selectivity, and high productivity stably for a prolonged period of time of, for example, not less than 1000 hours, preferably not less than 3000 hours, more preferably not less than 5000 hours, from the cyclic carbonate and the aliphatic monohydric alcohol. The reason why it has become possible to produce the dialkyl carbonate and the diol on an industrial scale with such excellent effects by implementing the process of the present invention is not clear, but this is supposed to be due to a composite effect brought about when the conditions of formulae (1) to (6), the conditions on the stages of the starting material inlets, and the conditions on the aperture ratios of the trays are combined.

The term "the aperture ratio of the tray" used in the present invention means, for each of the trays in the multi-stage distillation column, the ratio between the total area of openings in the tray through which gas and liquid can pass and the area of the tray having these openings therein. Note that for a tray having a downcomer portion, the area of the portion in which bubbling substantially occurs, i.e. excluding the downcomer portion, is taken as the area of the tray.

The present invention relates to a reactive distillation method in which not only distillation is carried out but rather reaction is carried out at the same time, and a high conversion and high selectivity (high yield) are attained; to achieve this, it has been discovered that in addition to the above formulae (1) to (6) being satisfied, it is important to make the stages of the starting material inlets and the aperture ratios of the tray be in the above specified ranges.

Preferable ranges for the respective factors are described below.

If L (cm) is less than 2100, then the conversion decreases and hence it is not possible to attain the desired production amount. Moreover, to keep down the equipment cost while securing a conversion enabling the desired production amount to be attained, L must be made to be not more than 8000. A more preferable range for L (cm) is $2300 \leq L \leq 6000$, with $2500 \leq L \leq 5000$ being yet more preferable.

If D (cm) is less than 180, then it is not possible to attain the desired production amount. Moreover, to keep down the equipment cost while attaining the desired production amount, D must be made to be not more than 2000. A more preferable range for D (cm) is $200 \leq D \leq 1000$, with $210 \leq D \leq 800$ being yet more preferable.

If L / D is less than 4 or greater than 40, then stable operation becomes difficult. In particular, if L / D is greater than 40, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. A more preferable range for L / D is $5 \leq L / D \leq 30$, with $7 \leq L / D \leq 20$ being yet more preferable.

If n is less than 10, then the conversion decreases and hence it is not possible to attain the desired production amount. Moreover, to keep down the equipment cost while securing the conversion enabling the desired production amount to be attained, n must be made to be not more than 120. Furthermore, if n is greater than 120, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. A more preferable range for n is $30 \leq n \leq 100$, with $40 \leq n \leq 90$ being yet more preferable.

If $D / d_1$ is less than 3, then the equipment cost becomes high. Moreover, a large amount of a gaseous component is readily released to the outside of the system, and hence stable operation becomes difficult. If $D / d_1$ is greater than 20, then the gaseous component withdrawal amount becomes relatively low, and hence stable operation becomes difficult, and moreover a decrease in the conversion is brought about. A more preferable range for $D / d_1$ is $4 \leq D / d_1 \leq 15$, with $5 \leq D / d_1 \leq 13$ being yet more preferable.

If $D / d_2$ is less than 5, then the equipment cost becomes high. Moreover, the liquid withdrawal amount becomes relatively high, and hence stable operation becomes difficult. If $D / d_2$ is greater than 30, then the flow rate through the liquid outlet and piping becomes excessively fast, and hence erosion becomes liable to occur, bringing about corrosion of the apparatus. A more preferable range for $D / d_2$ is $7 \leq D / d_2 \leq 25$, with $9 \leq D / d_2 \leq 20$ being yet more preferable.

Furthermore, it has been found in the present invention that it is still more preferable if $d_1$ and $d_2$ satisfy the formula (7);

$$1 \leq d_1 / d_2 \leq 5 \qquad (7).$$

In the present invention, the trays must have aperture ratios in specified ranges. Specifically, the aperture ratio of the trays installed not lower than the stage where the cyclic carbonate is introduced must be in a range of from 2.5 to 10%, the aperture ratio of the trays installed not higher than the stage where the cyclic carbonate is introduced and not lower than the stage where the aliphatic monohydric alcohol is introduced must be in a range of from 2 to 8%, and the aperture ratio of the trays installed not higher than the stage where the aliphatic monohydric alcohol is introduced must be in a range of from 1.5 to 5%. If one of these aperture ratios is less than the specified lower limit, then the apparatus becomes large relative to the required production amount, and hence the equipment cost becomes high. Moreover, the residence time increases, and hence side reactions (e.g. a reaction between the reaction product diol and unreacted cyclic carbonate) become liable to occur. Moreover, if one of these aperture ratios is greater than the specified upper limit, then the residence time in each tray decreases, and hence the number of stages must be increased to attain a high conversion, and thus the problems described above for when n is large arise.

For such reasons, the aperture ratio of the trays installed not lower than the stage where the cyclic carbonate is introduced is preferably in a range of from 3 to 8%, more preferably from 3.5 to 6%. Moreover, the aperture ratio of the trays installed not higher than the stage where the cyclic carbonate is introduced and not lower than the stage where the aliphatic monohydric alcohol is introduced is preferably in a range of from 2.5 to 7%, more preferably from 3 to 5.5%. Furthermore, the aperture ratio of the trays installed not higher than the stage where the aliphatic monohydric alcohol is introduced is preferably in a range of from 1.8 to 4.5%, more preferably from 2 to 4%.

In the present invention, each tray must have at least three types of aperture ratio, being in three zones divided from one another by the stages of the inlets for the two starting materials. In each zone, the aperture ratio may be the same for all of the trays, or may differ. In the case of differing, the aperture ratio of trays in an upper portion of the zone is preferably greater than the aperture ratio of trays in a lower portion of the zone.

In the present invention, the aperture ratio of the trays in a zone in an upper portion of the column is preferably the same as or greater than the aperture ratio of the trays in a zone in a lower portion of the column. A multi-stage distillation column is preferably used. It is thus particularly preferable for the following relationship to be satisfied: aperture ratio in upper portion ≥ aperture ratio in middle portion ≥ aperture ratio in lower portion.

The term "prolonged stable operation" used in the present invention means that operation can be carried out continuously in a steady state based on the operating conditions with no flooding, weeping, clogging of piping, or erosion for not less than 1000 hours, preferably not less than 3000 hours, more preferably not less than 5000 hours, and predetermined amounts of the dialkyl carbonate and the diol can be produced while maintaining a high conversion, high selectivity, and high productivity.

A characteristic feature of the present invention is that the dialkyl carbonate and the diol can be produced stably for a prolonged period of time each with high selectivity and preferably with high productivity for the dialkyl carbonate of not less than 2 ton / hr and high productivity for the diol of not less than 1.3 ton / hr. The dialkyl carbonate and the diol are more preferably produced in an amount of not less than 3 ton / hr and not less than 1.95 ton / hr respectively, yet more preferably not less than 4 ton / hr and not less than 2.6 ton / hr respectively. Moreover, another characteristic feature of the present invention is that in the case that L, D, L / D, n, D / $d_1$, and D / $d_2$ for the continuous multi-stage distillation column satisfy respectively $2300 \leq L \leq 6000$, $200 \leq D \leq 1000$, $5 \leq L/D \leq 30$, $30 \leq n \leq 100$, $4 \leq D/d_1 \leq 15$, and $7 \leq D / d_2 \leq 25$, not less than 2.5 ton / hr, preferably not less than 3 ton / hr, more preferably not less than 3.5 ton / hr of the dialkyl carbonate, and not less than 1.6 ton / hr, preferably not less than 1.95 ton / hr, more preferably not less than 2.2 ton / hr of the diol can be produced. Furthermore, another characteristic feature of the present invention is that in the case that L, D, L / D, n, D / $d_1$, and D / $d_2$ for the continuous multi-stage distillation column satisfy respectively $2500 \leq L \leq 5000$, $210 \leq D \leq 800$, $7 \leq L / D \leq 20$, $40 \leq n \leq 90$,$5 \leq D / d_1 \leq 13$, and $9 \leq D / d_2 \leq 20$, not less than 3 ton / hr, preferably not less than 3.5 ton / hr, more preferably not less than 4 ton / hr of the dialkyl carbonate, and not less than 1.95 ton / hr, preferably not less than 2.2 ton / hr, more preferably not less than 2.6 ton / hr of the diol can be produced.

The "selectivity" for each of the dialkyl carbonate and the diol in the present invention is based on the cyclic carbonate reacted. In the present invention, a high selectivity of not less than 95% can generally be attained, preferably not less than 97%, more preferably not less than 99%. Moreover, the "conversion" in the present invention generally indicates the cyclic carbonate conversion, in the present invention it being possible to make the cyclic carbonate conversion be not less than 95%, preferably not less than 97%, more preferably not less than 99%, yet more preferably not less than 99.5%, still more preferably not less than 99.9%. It is one of the excellent characteristic features of the present invention that a high conversion can be maintained while maintaining high selectivity in this way.

The continuous multi-stage distillation column used in the present invention comprises a tray column type distillation column including trays having a plurality of holes as the internal. The term "internal" used in the present invention means the parts in the distillation column where gas and liquid are actually brought into contact with one another. Examples of the tray include a bubble-cap tray, a sieve tray, a ripple tray, a ballast tray, a valve tray, a counterflow tray, an Unifrax tray, a Superfrac tray, a Maxfrac tray, a dual flow tray, a grid plate tray, a turbogrid plate tray, a Kittel tray, or the like. In the case that there are stages in the continuous multi-stage distillation column in which the catalyst is not present and hence reaction substantially does not take place (e.g. stages above the stage at which the catalyst is introduced), a distillation column in which these stages are packed with packing, i.e. a multi-stage distillation column having both a tray portion and a portion packed with the packing, is also preferable. Examples of the packing include a random packing such as a Raschig ring, a Lessing ring, a Pall ring, a Berl saddle, an Intalox saddle, a Dixon packing, a McMahon packing or Heli-Pak, or a structured packing such as Mellapak, Gempak, Techno-pack, Flexipac, a Sulzer packing, a Goodroll packing or Glitschgrid. Furthermore, the term "number of stages n" used in the present invention means the number of trays in the case of the tray, and the theoretical number of stages in the case of the packing. The number of stages n in the case of a multi-stage distillation column having both a tray portion and a portion packed with the packing is thus the sum of the number of trays and the theoretical number of stages.

For the process of the present invention, it has been discovered that a high conversion, high selectivity, and high productivity can be attained if n stages of any of the above trays are used, but that sieve trays each having a sieve portion and a downcomer portion are particularly good as the trays in terms of the relationship between performance and equipment cost. It has also been discovered that each sieve tray preferably has 100 to 1000 holes / $m^2$ in the sieve portion. A more preferable number of holes is 120 to 900 holes / $m^2$, yet more preferably 150 to 800 holes / $m^2$. Moreover, it has been discovered that the cross-sectional area per hole of each sieve tray is preferably in a range of from 0.5 to 5 $cm^2$ . A more preferable cross-sectional area per hole is from 0.7 to 4 $cm^2$, yet more preferably from 0.9 to 3 $cm^2$. Furthermore, it has been discovered that it is particularly preferable if each sieve tray has 100 to 1000 holes / $m^2$ in the sieve portion, and the cross-sectional area per hole is in a range of from 0.5 to 5 $cm^2$. The number of holes in the sieve portion may be the same for all of the sieve trays, or may differ.

It has been shown that by adding the above conditions to the continuous multi-stage distillation column, the object of the present invention can be attained more easily.

When carrying out the present invention, the dialkyl carbonate and the diol are continuously produced by continuously feeding the cyclic carbonate and the aliphatic monohydric alcohol as the starting materials into the continuous multi-stage distillation column in which the catalyst is present, carrying out reaction and distillation simultaneously in the column, continuously withdrawing a low boiling point reaction mixture containing the produced dialkyl carbonate from an upper portion of the column in a gaseous form, and continuously withdrawing a high boiling point reaction mixture containing the diol from a lower portion of the column in a liquid form.

The reaction time for the transesterification reaction carried out in the present invention is considered to equate to the average residence time of the reaction liquid in the continuous multi-stage distillation column. The reaction time varies depending on the form of the internals in the distillation column and the number of stages, the amounts of the starting materials fed in, the type and amount of the catalyst, the reaction conditions, and so on. The reaction time is

generally in a range of from 0.1 to 20 hours, preferably from 0.5 to 15 hours, more preferably from 1 to 10 hours.

The reaction temperature varies depending on the type of the starting material compounds used, and the type and amount of the catalyst. The reaction temperature is generally in a range of from 30 to 300 °C. It is preferable to increase the reaction temperature so as to increase the reaction rate. However, if the reaction temperature is too high, then side reactions become liable to occur. The reaction temperature is thus preferably in a range of from 40 to 250 °C, more preferably from 50 to 200 °C, yet more preferably from 60 to 150 °C. In the present invention, the reactive distillation can be carried out with the column bottom temperature set to not more than 150 °C, preferably not more than 130 °C, more preferably not more than 110 °C, yet more preferably not more than 100 °C. An excellent characteristic feature of the present invention is that a high conversion, high selectivity, and high productivity can be attained even with such a low column bottom temperature. Moreover, the reaction pressure varies depending on the type of the starting material compounds used and the composition therebetween, the reaction temperature, and so on. The reaction pressure may be any of a reduced pressure, normal pressure, or an applied pressure, and is generally in a range of from 1 to $2x10^7$ Pa, preferably from $10^3$ to $10^7$ Pa, more preferably from $10^4$ to $5x10^6$ Pa.

The material constituting the continuous multi-stage distillation column used in the present invention is generally a metallic material such as carbon steel or stainless steel. In terms of the quality of the dialkyl carbonate and the diol to be produced, stainless steel is preferable.

EXAMPLE

Following is a more detailed description of the present invention through examples. However, the present invention is not limited to the following examples.

Example 1:

<Continuous multi-stage distillation column>

A continuous multi-stage distillation column as shown in FIG. 1 having L = 3300 cm, D = 300 cm, L / D = 11, n = 60, $D/d_1$ = 7.5, and $D / d_2$ = 12 was used. The trays in the distillation column were sieve trays, each having the cross-sectional area per hole in the sieve portion thereof of approximately 1.3 $cm^2$. The aperture ratio of each of the trays above a cyclic carbonate inlet was in a range of from 3.6 to 4.6%, the aperture ratio of each of the trays installed not higher than the stage where the cyclic carbonate is introduced and not lower than a stage where the aliphatic monohydric alcohol is introduced was in a range of from 2.6 to 3.6%, and the aperture ratio of each of the trays installed not higher than the stage where the aliphatic monohydric alcohol is introduced was in a range of from 1.6 to 2.6%.

<Reactive distillation>

3.27 Ton / hr of ethylene carbonate in a liquid form was continuously introduced into the distillation column from an inlet (3-a) provided at the $5^{th}$ stage from the top. 3.238 Ton / hr of methanol in a gaseous form (containing 8.96 % by weight of dimethyl carbonate) and 7.489 ton / hr of methanol in a liquid form (containing 6.66 % by weight of dimethyl carbonate) were respectively continuously introduced into the distillation column from inlets (3-b and 3-c) provided at the $30^{th}$ stage from the top. The molar ratio of the starting materials introduced into the distillation column was methanol / ethylene carbonate = 8.36.

The catalyst used was obtained by adding 4.8 ton of ethylene glycol to 2.5 ton of KOH (48 % by weight aqueous solution), heating to approximately 130 °C, gradually reducing the pressure, and carrying out heat treatment for approximately 3 hours at approximately 1300 Pa, so as to produce a homogeneous solution. This catalyst solution was continuously introduced into the distillation column from an inlet (3-e) provided at the $54^{th}$ stage from the bottom (K concentration: 0.1 % by weight based on ethylene carbonate fed in). Reactive distillation was carried out continuously under conditions of a column bottom temperature of 98 °C, a column top pressure of approximately $1.118x10^5$ Pa, and a reflux ratio of 0.42.

It was possible to attain stable steady state operation after 24 hours. A low boiling point reaction mixture withdrawn from the top 1 of the column in a gaseous form was cooled using a heat exchanger and thus turned into a liquid. The liquid low boiling point reaction mixture, which was continuously withdrawn from the distillation column at 10.678 ton / hr, contained 4.129 ton / hr of dimethyl carbonate, and 6.549 ton / hr of methanol. A liquid continuously withdrawn from the bottom 2 of the column at 3.382 ton / hr contained 2.356 ton / hr of ethylene glycol, 1.014 ton / hr of methanol, and 4 kg / hr of unreacted ethylene carbonate. Excluding the dimethyl carbonate contained in the starting material, the actual produced amount of dimethyl carbonate was 3.340 ton / hr, and excluding the ethylene glycol contained in the catalyst solution, the actual produced amount of ethylene glycol was 2.301 ton / hr. The ethylene carbonate conversion was 99.88%, the dimethyl carbonate selectivity was not less than 99.99%, and the ethylene glycol

selectivity was not less than 99.99%.

Prolonged continuous operation was carried out under these conditions. After 500 hours, 2000 hours, 4000 hours, 5000 hours, and 6000 hours, the actual produced amounts per hour were 3.340 ton, 3.340 ton, 3.340 ton, 3.340 ton, and 3.340 ton respectively for dimethyl carbonate, and 2.301 ton, 2.301 ton, 2.301 ton, 2.301 ton, and 2.301 ton respectively for ethylene glycol, the ethylene carbonate conversions were respectively 99.90%, 99.89%, 99.89%, 99.88%, and 99.88%, the dimethyl carbonate selectivities were respectively not less than 99.99%, not less than 99.99%, not less than 99.99%, not less than 99.99%, and not less than 99.99%, and the ethylene glycol selectivities were respectively not less than 99.99%, not less than 99.99%, not less than 99.99%, not less than 99.99%, and not less than 99.99%.

Example 2:

Reactive distillation was carried out under the following conditions using the same continuous multi-stage distillation column as in Example 1. 2.61 Ton / hr of ethylene carbonate in a liquid form was continuously introduced into the distillation column from the inlet (3-a) provided at the 5$^{th}$ stage from the top. 4.233 Ton / hr of methanol in a gaseous form (containing 2.41 % by weight of dimethyl carbonate) and 4.227 ton / hr of methanol in a liquid form (containing 1.46 % by weight of dimethyl carbonate) were respectively continuously introduced into the distillation column from the inlets (3-b and 3-c) provided at the 30$^{th}$ stage from the top. The molar ratio of the starting materials introduced into the distillation column was methanol / ethylene carbonate = 8.73. The catalyst was made to be the same as in Example 1, and was continuously fed into the distillation column. Reactive distillation was carried out continuously under conditions of a column bottom temperature of 93 °C, a column top pressure of approximately $1.046 \times 10^5$ Pa, and a reflux ratio of 0.48.

It was possible to attain stable steady state operation after 24 hours. A low boiling point reaction mixture withdrawn from the top 1 of the column in a gaseous form was cooled using a heat exchanger and thus turned into a liquid. The liquid low boiling point reaction mixture, which was continuously withdrawn from the distillation column at 8.17 ton/hr, contained 2.84 ton / hr of dimethyl carbonate, and 5.33 ton / hr of methanol. A liquid continuously withdrawn from the bottom 2 of the column at 2.937 ton / hr contained 1.865 ton / hr of ethylene glycol, 1.062 ton / hr of methanol, and 0.2 kg / hr of unreacted ethylene carbonate. Excluding the dimethyl carbonate contained in the starting material, the actual produced amount of dimethyl carbonate was 2.669 ton / hr, and excluding the ethylene glycol contained in the catalyst solution, the actual produced amount of ethylene glycol was 1.839 ton / hr. The ethylene carbonate conversion was 99.99%, the dimethyl carbonate selectivity was not less than 99.99%, and the ethylene glycol selectivity was not less than 99.99%.

Prolonged continuous operation was carried out under these conditions. After 1000 hours, 2000 hours, 3000 hours, and 5000 hours, the actual produced amounts per hour were 2.669 ton, 2.669 ton, 2.669 ton, and 2.669 ton respectively for dimethyl carbonate, and 1.839 ton, 1.839 ton, 1.839 ton, and 1.839 ton respectively for ethylene glycol, the ethylene carbonate conversions were respectively 99.99%, 99.99%, 99.99%, and 99.99%, the dimethyl carbonate selectivities were respectively not less than 99.99%, not less than 99.99%, not less than 99.99%, and not less than 99.99%, and the ethylene glycol selectivities were respectively not less than 99.99%, not less than 99.99%, not less than 99.99%, and not less than 99.99%.

Example 3:

The continuous multi-stage distillation column as shown in FIG. 1 having L = 3300 cm, D = 300 cm, L / D = 11, n = 60, $D / d_1 = 7.5$, and $D / d_2 = 12$ was used. The trays in the distillation column were sieve trays, each having the cross-sectional area per hole in the sieve portion thereof of approximately 1.3 $cm^2$. The aperture ratio of each of the trays above a cyclic carbonate inlet was in a range of from 4.1 to 5.1 %, the aperture ratio of each of the trays installed not higher than the stage where the cyclic carbonate is introduced and not lower than a stage where the aliphatic monohydric alcohol is introduced was in a range of from 3.1 to 4.1 %, and the aperture ratio of each of the trays installed not higher than the stage where the aliphatic monohydric alcohol is introduced was in a range of from 2.1 to 3.1 %.

3.773 Ton / hr of ethylene carbonate in a liquid form was continuously introduced into the distillation column from the inlet (3-a) provided at the 5$^{th}$ stage from the top. 3.736 Ton / hr of methanol in a gaseous form (containing 8.97 % by weight of dimethyl carbonate) and 8.641 ton / hr of methanol in a liquid form (containing 6.65 % by weight of dimethyl carbonate) were respectively continuously introduced into the distillation column from the inlets (3-b and 3-c) provided at the 30$^{th}$ stage from the top. The molar ratio of the starting materials introduced into the distillation column was methanol / ethylene carbonate = 8.73. The catalyst was made to be the same as in Example 1, and was continuously fed into the distillation column. Reactive distillation was carried out continuously under conditions of a column bottom temperature of 98 °C, a column top pressure of approximately $1.118 \times 10^5$ Pa, and a reflux ratio of 0.42.

It was possible to attain stable steady state operation after 24 hours. A low boiling point reaction mixture withdrawn from the top of the column in a gaseous form was cooled using a heat exchanger and thus turned into a liquid. The liquid low boiling point reaction mixture, which was continuously withdrawn from the distillation column at 12.32 ton

/ hr, contained 4.764 ton / hr of dimethyl carbonate, and 7.556 ton / hr of methanol. A liquid continuously withdrawn from the bottom of the column at 3.902 ton / hr contained 2.718 ton / hr of ethylene glycol, 1.17 ton / hr of methanol, and 4.6 kg / hr of unreacted ethylene carbonate. Excluding the dimethyl carbonate contained in the starting material, the actual produced amount of dimethyl carbonate was 3.854 ton / hr, and excluding the ethylene glycol contained in the catalyst solution, the actual produced amount of ethylene glycol was 2.655 ton / hr. The ethylene carbonate conversion was 99.88%, the dimethyl carbonate selectivity was not less than 99.99%, and the ethylene glycol selectivity was not less than 99.99%.

Prolonged continuous operation was carried out under these conditions. After 1000 hours, 2000 hours, 3000 hours, and 5000 hours, the actual produced amounts per hour were 3.854 ton, 3.854 ton, 3.854 ton, and 3.854 ton respectively for dimethyl carbonate, and 2.655 ton, 2.655 ton, 2.655 ton, and 2.655 ton respectively for ethylene glycol, the ethylene carbonate conversions were respectively 99.99%, 99.99%, 99.99%, and 99.99%, the dimethyl carbonate selectivities were respectively not less than 99.99%, not less than 99.99%, not less than 99.99%, and not less than 99.99%, and the ethylene glycol selectivities were respectively not less than 99.99%, not less than 99.99%, not less than 99.99%, and not less than 99.99%.

Example 4:

The continuous multi-stage distillation column as shown in FIG. 1 having L = 3300 cm, D = 300 cm, L / D = 11, n = 60, D / $d_1$ = 7.5, and D / $d_2$ = 12 was used. The trays in the distillation column were sieve trays, each having the cross-sectional area per hole in the sieve portion thereof of approximately 1.3 $cm^2$. The aperture ratio of each of the trays above a cyclic carbonate inlet was in a range of from 4 to 5%, the aperture ratio of each of the trays installed not higher than the stage where the cyclic carbonate is introduced and not lower than a stage where the aliphatic monohydric alcohol is introduced was in a range of from 4 to 5%, and the aperture ratio of each of the trays installed not higher than the stage where the aliphatic monohydric alcohol is introduced was in a range of from 2.5 to 3.5%.

7.546 Ton / hr of ethylene carbonate in a liquid form was continuously introduced into the distillation column from the inlet (3-a) provided at the $5^{th}$ stage from the top. 7.742 Ton / hr of methanol in a gaseous form (containing 8.95 % by weight of dimethyl carbonate) and 17.282 ton / hr of methanol in a liquid form (containing 6.66 % by weight of dimethyl carbonate) were respectively continuously introduced into the distillation column from the inlets (3-b and 3-c) provided at the $30^{th}$ stage from the top. The molar ratio of the starting materials introduced into the distillation column was methanol / ethylene carbonate = 8.36. The catalyst was made to be the same as in Example 1, and was continuously fed into the distillation column. Reactive distillation was carried out continuously under conditions of a column top temperature of 65 °C, a column top pressure of approximately $1.118 \times 10^5$ Pa, and a reflux ratio of 0.42.

It was possible to attain stable steady state operation after 24 hours. A low boiling point reaction mixture withdrawn from the top 1 of the column in a gaseous form was cooled using a heat exchanger and thus turned into a liquid. The liquid low boiling point reaction mixture, which was continuously withdrawn from the distillation column at 24.641 ton / hr, contained 9.527 ton / hr of dimethyl carbonate, and 15.114 ton / hr of methanol. A liquid continuously withdrawn from the bottom 2 of the column at 7.804 ton / hr contained 5.436 ton / hr of ethylene glycol, 2.34 ton / hr of methanol, and 23 kg / hr of unreacted ethylene carbonate. Excluding the dimethyl carbonate contained in the starting material, the actual produced amount of dimethyl carbonate was 7.708 ton / hr, and excluding the ethylene glycol contained in the catalyst solution, the actual produced amount of ethylene glycol was 5.31 ton / hr. The ethylene carbonate conversion was 99.7%, the dimethyl carbonate selectivity was not less than 99.99%, and the ethylene glycol selectivity was not less than 99.99%.

Prolonged continuous operation was carried out under these conditions. After 1000 hours, the actual produced amount per hour was 7.708 ton for dimethyl carbonate, and 5.31 ton for ethylene glycol, the ethylene carbonate conversion was 99.8%, the dimethyl carbonate selectivity was not less than 99.99%, and the ethylene glycol selectivity was not less than 99.99%.

Industrial Applicability

According to the present invention, it has been discovered that a dialkyl carbonate and a diol can be produced each with a high selectivity of not less than 95%, preferably not less than 97%, more preferably not less than 99%, on an industrial scale of not less than 2 ton / hr, preferably not less than 3 ton / hr, more preferably not less than 4 ton / hr, for the dialkyl carbonate, and not less than 1.3 ton / hr, preferably not less than 1.95 ton / hr, more preferably not less than 2.6 ton / hr, for the diol, with a high yield stably for a prolonged period of time of not less than 1000 hours, preferably not less than 3000 hours, more preferably not less than 5000 hours, from a cyclic carbonate and an aliphatic monohydric alcohol.

**Claims**

1. A process for producing a dialkyl carbonate and a diol industrially with a high yield in which the dialkyl carbonate and the diol are continuously produced through a reactive distillation system of taking a cyclic carbonate and an aliphatic monohydric alcohol as starting materials, comprising the steps of:

   continuously feeding the starting materials into a continuous multi-stage distillation column in which a homogeneous catalyst is present;
   carrying out reaction and distillation simultaneously in the column;
   continuously withdrawing a low boiling point reaction mixture containing the produced dialkyl carbonate from an upper portion of said column in a gaseous form; and
   continuously withdrawing a high boiling point reaction mixture containing the diol from a lower portion of the column in a liquid form,

   wherein:

   (a) said continuous multi-stage distillation column comprises a tray column type distillation column having a cylindrical trunk portion having a length L (cm) and an inside diameter D (cm) and having a structure having an internal with a number of stages n, said internal being a tray having a plurality of holes, and further having a gas outlet having an inside diameter $d_1$ (cm) at the top of the column or in the upper portion of the column near to the top, a liquid outlet having an inside diameter $d_2$ (cm) at the bottom of the column or in the lower portion of the column near to the bottom, at least one first inlet provided in the upper portion and / or a middle portion of the column below the gas outlet, and at least one second inlet provided in the middle portion and / or the lower portion of the column above the liquid outlet, wherein:

   (1) the length L (cm) satisfies the formula (1);

$$2100 \le L \le 8000 \qquad (1),$$

   (2) the inside diameter D (cm) of the column satisfies the formula (2);

$$180 \le D \le 2000 \qquad (2),$$

   (3) a ratio of the length L (cm) to the inside diameter D (cm) of the column satisfies the formula (3);

$$4 \le L / D \le 40 \qquad (3),$$

   (4) the number of stages n satisfies the formula (4);

$$10 \le n \le 120 \qquad (4),$$

   (5) a ratio of the inside diameter D (cm) of the column to the inside diameter $d_1$ (cm) of the gas outlet satisfies the formula (5);

$$3 \le D / d_1 \le 20 \qquad (5),$$

   and
   (6) a ratio of the inside diameter D (cm) of the column to the inside diameter $d_2$ (cm) of the liquid outlet

satisfies the formula (6);

$$5 \leq D / d_2 \leq 30 \qquad (6);$$

(b) the starting material cyclic carbonate is continuously introduced into said continuous multi-stage distillation column from at least one said first inlet provided between the 3rd stage from the top of said continuous multi-stage distillation column and the $(n/3)^{th}$ stage from the top of said continuous multi-stage distillation column;
(c) the starting material aliphatic monohydric alcohol is continuously introduced into said continuous multi-stage distillation column from at least one said second inlet provided between the $(n/3)^{th}$ stage from the top of said continuous multi-stage distillation column and the $(2n/3)^{th}$ stage from the top of said continuous multi-stage distillation column;
(d) an aperture ratio of the tray installed not lower than a stage where the cyclic carbonate is introduced is in a range of from 2.5 to 10%;
(e) an aperture ratio of the tray installed not higher than the stage where the cyclic carbonate is introduced and not lower than a stage where the aliphatic monohydric alcohol is introduced is in a range of from 2 to 8%; and
(f) an aperture ratio of the tray installed not higher than the stage

where the aliphatic monohydric alcohol is introduced is in a range of from 1.5 to 5%.

**2.** The process according to Claim 1, wherein a produced amount of the dialkyl carbonate is not less than 2 ton / hr.

**3.** The process according to Claim 1 or 2, wherein the produced amount of the diol is not less than 1.3 ton / hr.

**4.** The process according to any one of Claims 1 to 3, wherein said $d_1$ and said $d_2$ satisfy the formula (7)

$$1 \leq d_1 / d_2 \leq 5 \qquad (7).$$

**5.** The process according to any one of Claims 1 to 4, wherein L, D, L / D, n, $D / d_1$, and $D / d_2$ for said continuous multi-stage distillation column satisfy respectively $2300 \leq L \leq 6000$, $200 \leq D \leq 1000$, $5 \leq L / D \leq 30$, $30 \leq n \leq 100$, $4 \leq D / d_1 \leq 15$, and $7 \leq D / d_2 \leq 25$.

**6.** The process according to any one of Claims 1 to 5, wherein L, D, L / D, n, $D / d_1$, and $D / d_2$ for said continuous multi-stage distillation column satisfy respectively $2500 \leq L \leq 5000$, $210 \leq D \leq 800$, $7 \leq L / D \leq 20$, $40 \leq n \leq 90$, $5 \leq D / d_1 \leq 13$, and $9 \leq D / d_2 \leq 20$.

**7.** The process according to any one of Claims 1 to 6, wherein the aperture ratio of the tray installed not lower than said stage where the cyclic carbonate is introduced is in a range of from 3 to 8%, the aperture ratio of the tray installed not higher than said stage where the cyclic carbonate is introduced and not lower than said stage where the aliphatic monohydric alcohol is introduced is in a range of from 2.5 to 7%, and the aperture ratio of the tray installed not higher than said stage where the aliphatic monohydric alcohol is introduced is in a range of from 1.8 to 4.5%.

**8.** The process according to any one of Claims 1 to 7, wherein the aperture ratio of the tray installed not lower than the stage where the cyclic carbonate is introduced is in a range of from 3.5 to 6%, the aperture ratio of the tray installed not higher than the stage where the cyclic carbonate is introduced and not lower than the stage where the aliphatic monohydric alcohol is introduced is in a range of from 3 to 5.5%, and the aperture ratio of the tray installed not higher than the stage where the aliphatic monohydric alcohol is introduced is in a range of from 2 to 4%.

**9.** The process according to any one of Claims 1 to 8, wherein said tray is a sieve tray having a sieve portion and a downcomer portion.

**10.** The process according to Claim 9, wherein said sieve trays has 100 to 1000 holes / $m^2$ in said sieve portion thereof.

**11.** The process according to Claim 9 or 10, wherein a cross-sectional area per hole of said sieve trays is in a range of

from 0.5 to 5 $cm^2$.

**12.** A continuous multi-stage distillation column for carrying out transesterification between a cyclic carbonate and an aliphatic monohydric alcohol and distillation, wherein:

(a) the continuous multi-stage distillation column comprises:

a cylindrical trunk portion having a length L (cm) and an inside diameter D (cm);
a tray having number of stages n provided inside the trunk portion;
a gas outlet having an inside diameter $d_1$ (cm) provided at a top of the column or in an upper portion of the column near to the top;
a liquid outlet having an inside diameter $d_2$ (cm) provided at a bottom of the column or in a lower portion of the column near to the bottom;
at least one first inlet provided in the upper portion and / or a middle portion of the column below said gas outlet; and
at least one second inlet provided in the middle portion and / or the lower portion of the column above said liquid outlet;

wherein:

(1) the length L (cm) satisfies the formula (1);

$$2100 \leq L \leq 8000 \qquad (1),$$

(2) the inside diameter D (cm) of the column satisfies the formula (2);

$$180 \leq D \leq 2000 \qquad (2),$$

(3) a ratio of the length L (cm) to the inside diameter D (cm) of the column satisfies the formula (3);

$$4 \leq L / D \leq 40 \qquad (3),$$

(4) the number of stages n satisfies the formula (4);

$$10 \leq n \leq 120 \qquad (4),$$

(5) a ratio of the inside diameter D (cm) of the column to the inside diameter $d_1$ (cm) of the gas outlet satisfies the formula (5);

$$3 \leq D / d_1 \leq 20 \qquad (5),$$

and
(6) a ratio of the inside diameter D (cm) of the column to the inside diameter $d_2$ (cm) of the liquid outlet satisfies the formula (6),

$$5 \leq D / d_2 \leq 30 \qquad (6);$$

(b) the starting material cyclic carbonate is continuously introduced into said continuous multi-stage distillation column from at least one said first inlet provided between the $3^{rd}$ stage from the top of said continuous multi-stage distillation column and the $(n/3)^{th}$ stage from the top of said continuous multi-stage distillation column;
(c) the starting material aliphatic monohydric alcohol is continuously introduced into said continuous multi-stage distillation column from at least one said second inlet provided between the $(n/3)^{th}$ stage from the top of said continuous multi-stage distillation column and the $(2n/3)^{th}$ stage from the top of said continuous multi-stage distillation column;
(d) an aperture ratio of the tray installed not lower than a stage where the cyclic carbonate is introduced is in a range of from 2.5 to 10%;
(e) an aperture ratio of the tray installed not higher than the stage where the cyclic carbonate is introduced and not lower than a stage where the aliphatic monohydric alcohol is introduced is in a range of from 2 to 8%; and
(f) an aperture ratio of the tray installed not higher than the stage

where the aliphatic monohydric alcohol is introduced is in a range of from 1.5 to 5%.

**13.** The continuous multi-stage distillation column according to Claim 12, wherein said $d_1$ and said $d_2$ satisfy the formula (7);

$$1 \leq d_1 / d_2 \leq 5 \qquad (7).$$

**14.** The continuous multi-stage distillation column according to Claim 12 or 13, wherein L, D, L / D, n, D / $d_1$, and D / $d_2$ for said continuous multi-stage distillation column satisfy respectively $2300 \leq L \leq 6000$, $200 \leq D \leq 1000$, $5 \leq L / D \leq 30$, $30 \leq n \leq 100$, $4 \leq D / d_1 \leq 15$, and $7 \leq D / d_2 \leq 25$.

**15.** The continuous multi-stage distillation column according to any one of Claims 12 to 14, wherein L, D, L / D, n, D / $d_1$, and D / $d_2$ for said continuous multi-stage distillation column satisfy respectively $2500 \leq L \leq 5000$, $210 \leq D \leq 800$, $7 \leq L / D \leq 20$,$40 \leq n \leq 90$,$5 \leq D / d_1 \leq 13$, and $9 \leq D / d_2 \leq 20$.

**16.** The continuous multi-stage distillation column according to any one of Claims 12 to 15, wherein the aperture ratio of the tray installed not lower than said stage where the cyclic carbonate is introduced is in a range of from 3 to 8%, the aperture ratio of the tray installed not higher than said stage where the cyclic carbonate is introduced and not lower than said stage where the aliphatic monohydric alcohol is introduced is in a range of from 2.5 to 7%, and the aperture ratio of the tray installed not higher than said stage where the aliphatic monohydric alcohol is introduced is in a range of from 1.8 to 4.5%.

**17.** The continuous multi-stage distillation column according to any one of Claims 12 to 16, wherein the aperture ratio of the tray installed not lower than said stage where the cyclic carbonate is introduced is in a range of from 3.5 to 6%, the aperture ratio of the tray installed not higher than said stage where the cyclic carbonate is introduced and not lower than said stage where the aliphatic monohydric alcohol is introduced is in a range of from 3 to 5.5%, and the aperture ratio of the trays installed not higher than said stage where the aliphatic monohydric alcohol is introduced is in a range of from 2 to 4%.

**18.** The continuous multi-stage distillation column according to any one of Claims 12 to 17, wherein said tray is a sieve tray having a sieve portion and a downcomer portion.

**19.** The continuous multi-stage distillation column according to Claim 18, wherein said sieve tray has 100 to 1000 holes / $m^2$ in said sieve portion thereof.

**20.** The continuous multi-stage distillation column according to Claim 18 or 19, wherein a cross-sectional area per hole of said sieve tray is in a range of from 0.5 to 5 $cm^2$.

# FIG.1

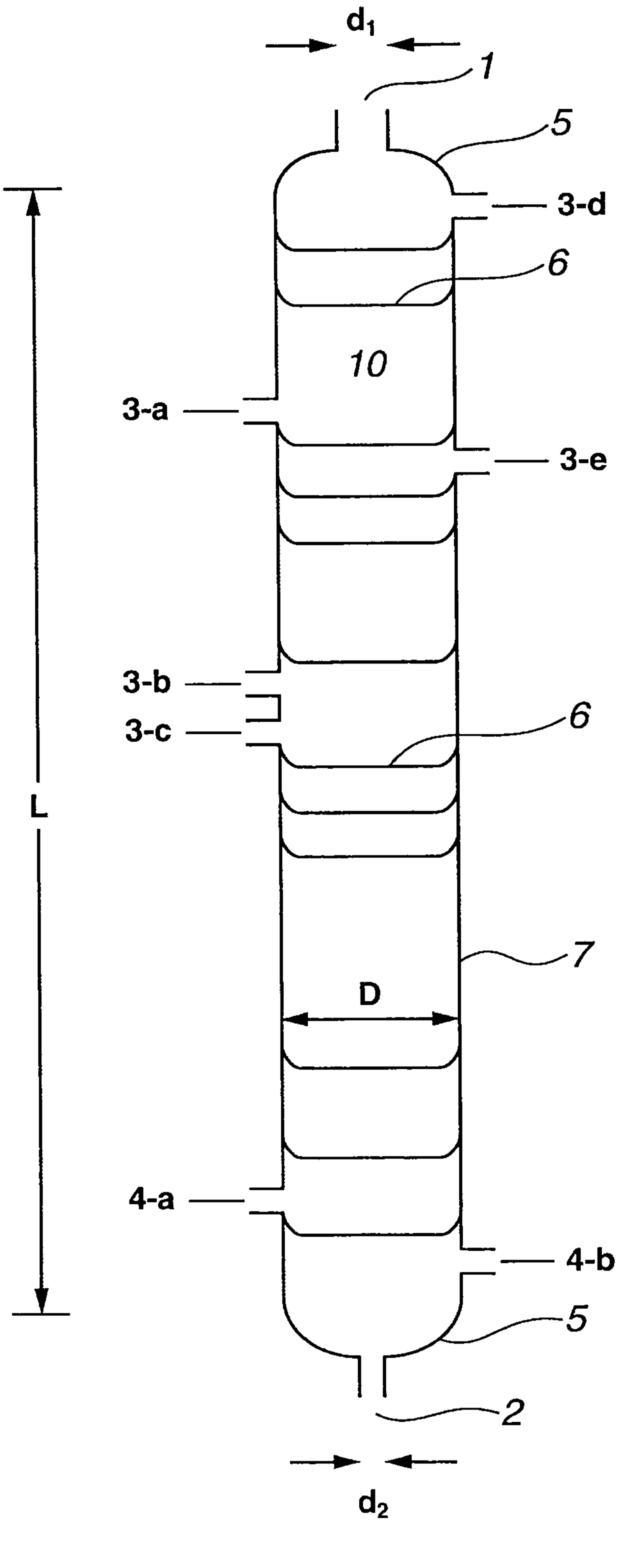

## INTERNATIONAL SEARCH REPORT

International application No. PCT/JP2006/324355

**A. CLASSIFICATION OF SUBJECT MATTER**

*C07C27/02*(2006.01)i, *B01D3/14*(2006.01)i, *B01D3/22*(2006.01)i, *B01D3/32*(2006.01)i, *C07C29/128*(2006.01)i, *C07C31/20*(2006.01)i, *C07C68/06*(2006.01)i, *C07C69/96*(2006.01)i, *C07B61/00*(2006.01)n

According to International Patent Classification (IPC) or to both national classification and IPC

**B. FIELDS SEARCHED**

Minimum documentation searched (classification system followed by classification symbols)

C07C27/02, B01D3/14, B01D3/22, B01D3/32, C07C29/128, C07C31/20, C07C68/06, C07C69/96, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 00/51954 A1 (Asahi Chemical Industry Co., Ltd.), 08 September, 2000 (08.09.00), & EP 1174406 A1 & US 6479689 B1 | 1-20 |
| A | JP 9-183744 A (Asahi Chemical Industry Co., Ltd.), 15 July, 1997 (15.07.97), (Family: none) | 1-20 |
| A | JP 2003-342209 A (Mitsubishi Chemical Corp.), 03 December, 2003 (03.12.03), (Family: none) | 1-20 |

☒ Further documents are listed in the continuation of Box C.

☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 31 January, 2007 (31.01.07) | 13 February, 2007 (13.02.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/324355

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 2005/123638 A1 (Asahi Kasei Chemicals Corp.), 29 December, 2005 (29.12.05), (Family: none) | 1-20 |
| P,A | WO 2006/001256 A1 (Asahi Kasei Chemicals Corp.), 05 January, 2006 (05.01.06), (Family: none) | 1-20 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3642858 A
- JP S5448715 B
- US 4181676 A
- JP S5463023 B
- JP S54148726 B
- JP S51122025 B
- US 4062884 A
- JP S5448716 B
- US 4307032 A
- US 3803201 A
- JP 63041432 A
- US 4661609 A
- US 4734518 A
- JP S63238043 B
- JP S6431737 B
- US 4691041 A
- JP S6341432 B
- JP H4198141 B
- JP H4230243 B
- JP H9176061 B
- JP H9183744 B
- JP H9194435 B
- WO 9723445 A
- JP 0889025 A
- US 5847189 A
- WO 9964382 A
- JP 1086940 A
- US 6346638 B
- WO 0051954 A
- EP 1174406 A
- US 6479689 B
- JP 2002308804 A
- JP 2004131394 A
- JP H5213830 B
- EP 0530615 A
- US 5231212 A
- EP H69507 A
- EP 0569812 A
- US 5359118 A
- EP 2003119168 A
- WO 03006418 A
- EP 2003300936 A
- EP 2003342209 A
- JP 2003300936 A